(19) Europäisches Patentamt
European Patent Office
Office européen des brevets

(11) Publication number: **0 021 848**
**B1**

(12) **EUROPEAN PATENT SPECIFICATION**

(45) Date of publication of patent specification: **13.04.83**

(51) Int. Cl.[3]: **C 07 C 37/08**, C 07 C 39/08

(21) Application number: **80302199.7**

(22) Date of filing: **30.06.80**

(54) **Process and apparatus for the preparation of phenols.**

(30) Priority: **30.06.79 JP 82019/79**

(43) Date of publication of application:
**07.01.81 Bulletin 81/1**

(45) Publication of the grant of the patent:
**13.04.83 Bulletin 83/15**

(84) Designated Contracting States:
**DE FR GB IT NL**

(56) References cited:
GB - A - 910 735
GB - A - 1 498 995
US - A - 3 271 457
US - A - 3 365 375

**The file contains technical information submitted after the application was filed and not included in this specification**

(73) Proprietor: **MITSUI PETROCHEMICAL INDUSTRIES, LTD.**
**2-5, Kasumigaseki 3-chome Chiyoda-ku**
**Tokyo 100 (JP)**

(72) Inventor: **Hashimoto, Katsuhiko**
**2-7, 1-chome**
**Muronoki-cho, Iwakuni-shi, Yamaguchi-ken (JP)**
Inventor: **Nambu, Hirohiko**
**37-21, 6-chome**
**Hirata, Iwakuni-shi, Yamaguchi-ken (JP)**
Inventor: **Watari, Kazuhiro**
**213-3, Waki**
**Waki-machi, Kuga-gun, Yamaguchi-ken (JP)**
Inventor: **Mizuno, Kenichi**
**111-6, 2-chome Minami-Iwakuni-cho**
**Iwakuni-shi, Yamaguchi-ken (JP)**
Inventor: **Murakami, Tadateru**
**12-35, 2-chome**
**Shinmachi, Otake-shi, Hiroshima-ken (JP)**
Inventor: **Matsuoka, Yutaka**
**2-3, 1-chome**
**Muronoki-cho, Iwakuni-shi, Yamaguchi-ken (JP)**
Inventor: **Moriyama, Mitsutoshi**
**6-15, 1-chome Kichijoji-minami-cho**
**Musashino-shi, Tokyo (JP)**
Inventor: **Ohno, Norio**
**213-2, Waki**
**Waki-machi, Kuga-gun, Yamaguchi-ken (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 021 848 B1**

Courier Press, Leamington Spa, England

(74) Representative: **Myerscough, Philip Boyd et al, J.A.Kemp & Co. 14, South Square Gray's Inn London, WC1R 5EU (GB)**

# Process and apparatus for the preparation of phenols

The present invention relates to a process and apparatus for preparing phenols from α-hydroxyalkyl-substituted aromatic compounds and/or α,β-unsaturated alkyl-substituted aromatic compounds.

It is known that when an α-hydroxyalkyl-substituted aromatic compound (referred to as "aromatic alcohol" hereinafter) and/or an α,β-unsaturated alkyl-substituted aromatic compound (referred to as "aromatic unsaturated compound" hereinafter) is reacted in a reaction vessel with hydrogen peroxide in the presence of an acidic catalyst, a phenol is formed (see, for example, British Patent Specification No. 910,735). Since such aromatic alcohols or aromatic unsaturated compounds are formed as by-products when alkyl aromatic compounds are oxidized to the corresponding hydroperoxides with molecular oxygen, when phenols are to be prepared by the acid decomposition of the hydroperoxides, the yield based on a starting alkyl aromatic compound will be increased if such by-products can be converted to the phenol. Accordingly, a process utilizing the above-mentioned reaction with hydrogen peroxide is economically attractive. However, hydrogen peroxide is a strong oxidant and any excess tends to react with the desired phenol, resulting in a reduction in the yield of the phenol and/or in a reduction in the quality of the phenol due to formation of colored impurities. In particular, when the volume of the reaction vessel is increased, it becomes difficult to carry out the intended reaction efficiently while controlling undesired side reactions.

The invention provides a process for preparing phenols of high quality in high yields and safely overcomes the defects associated with known processes.

In accordance with the present invention, there is provided a process for the preparation of phenols by reacting in a reaction vessel (1) an α-hydroxyalkyl-substituted aromatic compound and/or an α,β-unsaturated alkyl-substituted aromatic compound with hydrogen peroxide in the presence of an acid catalyst and, as solvent, acetone, characterised in that the reaction is carried out under such conditions that acetone is distilled off, condensed and recycled to the reaction vessel (1), hydrogen peroxide is diluted with at least 10 times its volume of recycled acetone, and the diluted hydrogen peroxide is scattered into the reaction vessel by sprinkling, the hydrogen peroxide being used in an amount smaller than the amount equimolar to the sum of the amount(s) of secondary alcoholic hydroxyl groups, tertiary alcoholic hydroxyl groups and/or olefinic double bonds in the α-hydroxyalkyl-substituted aromatic compound and/or the α,β-unsaturated alkyl-substituted aromatic compound.

Typical α-hydroxyalkyl-substituted aromatic compounds (or aromatic alcohols) that can be used as a starting material in the process of the present invention are represented by the general formula:

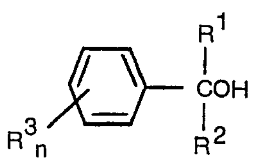

wherein $R^1$ and $R^2$ each independently represent a hydrogen atom or an alkyl group, preferably an alkyl group having 1 to 3 carbon atoms, $R^3$ represents a hydrogen atom, an alkyl group,

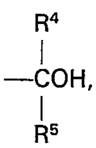 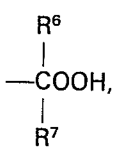 a halogen atom, —OH, 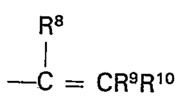 or 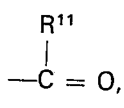

in which $R^4$, $R^5$, $R^6$, $R^7$, $R^8$, $R^9$ and $R^{10}$ each independently represent a hydrogen atom or an alkyl group and $R^{11}$ represents an alkyl group, and n is an integer of from 1 to 5 with the proviso that when n is 2 or more the groups $R^3$ may be the same or different.

As specific examples of such aromatic alcohols, there can be mentioned α-methylbenzyl alcohol, α-ethylbenzyl alcohol, α,α-dimethylbenzyl alcohol, α-methyl-α-ethylbenzyl alcohol, α,α-diethylbenzyl alcohol, α-methyl-p-methylbenzyl alcohol, α-methyl-m-methylbenzyl alcohol, α,α-dimethyl-p-methylbenzyl alcohol, α,α-dimethyl-m-methylbenzyl alcohol, α,α-dimethyl-p-isopropylbenzyl alcohol, α,α-dimethyl-p-isopropylbenzyl alcohol, α,α-dimethyl-p-isopropenylbenzyl alcohol, α,α-dimethyl-m-isopropenylbenzyl alcohol, α,α'-dihydroxy-p-diisopropylbenzene, α,α'-dihydroxy-m-diisopropylbenzene, α,α-dimethyl-p-hydroxybenzyl alcohol, α,α-dimethyl-m-hydroxybenzyl alcohol, α,α-dimethyl-m-chlorobenzyl alcohol, α-hydroxy-α'-hydroperoxy-p-diisopropylbenzene and α-hydroxy-α'-hydroperoxy-m-diisopropylbenzene.

Typical α,β-unsaturated alkyl-substituted aromatic compounds that can be used in the process of the present invention are represented by the general formula:

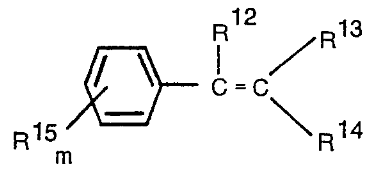

wherein $R^{12}$, $R^{13}$ and $R^{14}$ each independently represent a hydrogen atom or an alkyl group, $R^{15}$ represents a hydrogen atom, an alkyl group,

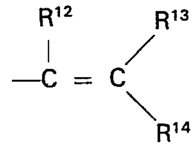 a halogen atom, 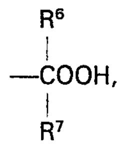 —OH or 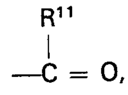

and m is an integer of from 1 to 5 with the proviso that when m is 2 or more the groups $R^{15}$ may be the same or different.

As specific examples of such aromatic unsaturated compounds, there can be mentioned α-methylstyrene, α-ethylstyrene, β-methylstyrene, isopropenyltoluene, isopropenylethylbenzene, isopropenylcumene, diisopropenylbenzene and isopropenylphenol. Mixtures of two or more of these compounds may be used.

In the present invention, a starting material containing the aromatic alcohol and/or aromatic unsaturated compound is used. The starting material may also contain other components. For example, the starting material may contain a hydroperoxide of an alkyl aromatic compound, such as cumene hydroperoxide, m-cymene hydroperoxide, p-cymene hydroperoxide, m-diisopropylbenzene monohydroperoxide, p-diisopropylbenzene monohydroperoxide, m-diisopropylbenzene dihydroperoxide or p-diisopropylbenzene dihydroperoxide. The starting material may also contain a hydrocarbon such as benzene, toluene, xylene, ethylbenzene, cumene, cymene or diisopropylbenzene and a ketone such as acetone, methylethyl ketone, diethyl ketone or methylisobutyl ketone. A reaction product obtained by oxidizing an alkyl aromatic compound with molecular oxygen and condensing the reaction mixture according to need contains not only a hydroperoxide of an alkyl aromatic compound but also an aromatic alcohol or aromatic unsaturated compound. This reaction product preferably is used as the starting material in the process of the present invention. When this starting material is reacted with hydrogen peroxide in an acid decomposition step, side reactions normally readily occur, but if the conditions of the present invention are adopted, the side reactions can be controlled and the intended reaction can be effectively carried out.

Various acid catalysts may be used in the present invention. For example, there can be mentioned inorganic acids such as sulfuric acid, phosphoric acid, perchloric acid and hydrochloric acid, solid acids such as silica-alumina, molybdic acid and phosphomolybdic acid, organic acids such as toluene-sulfonic acid and trichloroacetic acid, and ion exchange resins.

The amount of the acid catalyst used can vary according to the kind thereof and the water content in the starting reaction mixture, so that amounts cannot readily be given to cover all circumstances. However, if a liquid acid catalyst is employed, it is preferred that the amount of the acid catalyst be about 0.01 to about 10% by weight, especially about 0.1 to about 3.0% by weight, based on the reaction medium.

In the process of the present invention, by supplying hydrogen peroxide diluted with recycled acetone to the reaction system, the aromatic alcohol and/or aromatic unsaturated compound is converted to a corresponding phenol. The hydrogen peroxide is supplied to the reaction diluted with at least 10 times, preferably 15 to 100 times, its volume of recycled acetone. Acetone has a good compatibility with hydrogen peroxide and can be removed from the reaction product by evaporation. The reaction is carried out under reflux of acetone so as to remove the reaction heat.

From the viewpoint of safety, it is preferred to dilute the hydrogen peroxide just before it is supplied to the starting liquid mixture.

The diluted hydrogen peroxide is scattered into the reaction vessel by sprinkling. For example, there can be adopted a method in which a perforated plate is disposed in the upper portion of the reaction vessel and the diluted hydrogen peroxide is supplied and sprinkled through this perforated plate.

The amount of hydrogen peroxide used is smaller than the amount equimolar to the sum of the amount(s) of secondary alcoholic hydroxyl groups, tertiary alcoholic hydroxyl groups and/or olefinic double bonds in the α-hydroxyalkyl-substituted aromatic compound and/or α,β-unsaturated alkyl-substituted aromatic compound. If the amount of hydrogen peroxide exceeds this level, the excess hydrogen peroxide tends to oxidize the formed phenol or ketone, resulting in a reduction in the efficiency of utilization of hydrogen peroxide or the occurrence of undesired phenomena such as a reduction in the purity of the formed phenol.

The reaction is ordinarily carried out under agitation. The reaction temperature is ordinarily in the range of 20 to 100°C. and preferably in the range of 40 to 90°C. It is preferred that the reaction be conducted in a continuous manner, and the residence time is ordinarily in the range of 1 minute to 2 hours and preferably in the range of 5 minutes to 1 hour.

In accordance with one preferred embodiment of the present invention, there is provided a process for the preparation of phenols, which comprises the steps of (i) supplying a starting material containing a hydroperoxide of an isopropyl aromatic compound and an $\alpha$-hydroxypropyl-substituted aromatic compound and/or $\alpha,\beta$-unsaturated isopropenyl-substituted aromatic compound, which has been obtained by oxidizing an isopropyl aromatic compound, especially diisopropylbenzene or cymene, with molecular oxygen, to a liquid phase reaction mixture in a reaction vessel containing an acid catalyst and acetone by scattering the starting material from a first scattering device located in the gas phase above said reaction mixture, (ii) supplying diluted hydrogen peroxide, which has been formed by diluting hydrogen peroxide with acetone so that the volume is 10 to 100 times the volume of the undiluted hydrogen peroxide, into said reaction mixture by scattering into said reaction mixture from a second scattering device located in the gas phase above said reaction mixture, (iii) contacting the starting material, hydrogen peroxide and acid catalyst with one another while distilling off acetone from the reaction mixture, (iv) condensing the distilled acetone and recycling the condensed acetone as a diluent to step (ii), and (v) recovering the resulting phenol from the reaction mixture.

In this embodiment, it is preferred to locate the second scattering device at a position higher than the position of the first scattering device, and to scatter the diluted hydrogen peroxide and the starting material continuously through said second scattering device and said first scattering device, respectively.

For example, a scattering device as disclosed in U.S. Patent Specification No. 3,271,457 can be used as the second scattering device. In the process disclosed in this U.S. Patent Specification, an alkaryl hydroperoxide is mixed with acetone and the mixture is scattered into the reaction system. Accordingly, the process of the present invention is different from this known process in the point where hydrogen peroxide is diluted with acetone and the dilution is scattered independently from the starting aromatic compound.

From the viewpoints of the efficiency of utilization of hydrogen peroxide and the operation safety, in the present invention, it is most advantageous and preferred to use as second scattering device a scattering device comprising an annular acetone feed pipe having an opening in the lower portion thereof and a plurality of hydrogen peroxide feed nozzles, each nozzle having a discharge end located at substantially the same position as said opening, whereby hydrogen peroxide falling down from said nozzles is diluted with acetone.

The invention will now be described further by reference to the accompanying drawings, in which Fig. 1 is a diagram illustrating one embodiment of a reaction vessel for use in carrying out the process of the present invention, Fig. 2 is a diagram illustrating another embodiment of the reaction vessel, and Figs. 3 and 4 are diagrams illustrating a scattering device for introducing diluted hydrogen peroxide into the reaction vessel.

Figs. 1 and 2 will be explained by reference to a process in which an oxidation product of p-diisopropylbenzene is acid-decomposed in acetone as the solvent in the presence of hydrogen peroxide.

Referring to Fig. 1, an oxidation product of p-diisopropylbenzene is continuously supplied from a scattering device 2 located in the upper portion of a reaction vessel 1 through a pipe 3. Sulfuric acid acting as the catalyst is continuously supplied from a pipe 4 and acetone acting as solvent is continuously supplied from a pipe 5. The reaction heat is removed by evaporation of acetone, and evaporated acetone is condensed in a condenser 6 and then stored in a drum 7. Acetone from drum 7 is introduced through a pipe 8 and mixed with hydrogen peroxide introduced into the pipe 8 through a pipe 9. The mixture is scattered from a scattering device 10.

In this embodiment, it is preferred that the scattering devices 2 and 10 be of different diameter as shown in Figs. 1 and 2, and it is especially preferred that the diameter of the scattering device 2 be smaller than the diameter of the scattering device 10. In this case, mingling of the diluted hydrogen peroxide with the stream of the oxidation product of p-diisopropylbenzene is substantially prevented in the gas phase.

The embodiment shown in Fig. 2 differs from the embodiment shown in Fig. 1 only in the nature of the second scattering device 12.

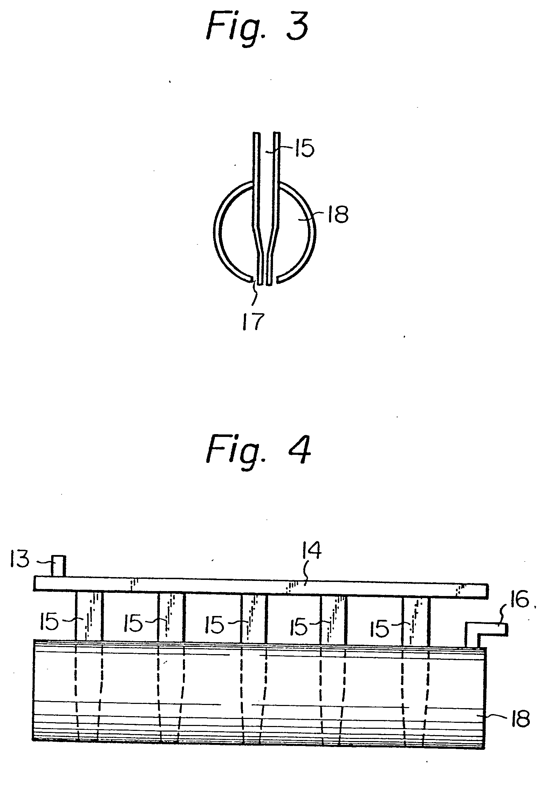

Figs. 3 and 4 are diagrams illustrating in detail the second scattering device 12 of Fig. 2. Hydrogen peroxide is introduced into a hydrogen peroxide-scattering annular pipe 14 from a pipe 13 and supplied in separate portions through a plurality of scattering nozzles 15. Refluxed acetone is supplied to an acetone-supplying annular pipe 18 having an opening 17 in the lower portion thereof from a pipe 16. The top end of each nozzle 15 communicates with the hydrogen peroxide feed pipe 14, while the discharge end is located at the position of the opening 17 of the acetone feed pipe 18, so that when hydrogen peroxide falls down from the scattering nozzles 15, it is diluted with a large quantity of acetone. If this scattering device is employed, hydrogen peroxide is diluted just before it enters the reaction vessel, and the phenol-forming reaction can be conducted very safely.

The present invention will now be described in detail by reference to the following Examples.

Example 1

A reaction vessel as shown in Fig. 2, which had an inner capacity of 50 litres, was used. A methylisobutyl ketone (MIBK) solution of an oxidation product of p-diisopropylbenzene, which contained 24% by weight of $\alpha,\alpha'$-dihydroperoxy-p-diisopropylbenzene (p-DHP) and 9.5% by weight of $\alpha$-hydroperoxy-$\alpha'$-hydroxy-p-diisopropylbenzene (p-HHP), was supplied into the reaction vessel at a rate of 35 l/hr. from a perforated plate 2 through a pipe 3. A 60% aqueous solution of hydrogen peroxide was supplied into the reaction vessel at a rate of 0.53 l/hr. (0.66 Kg/hr) from a scattering device 12 through a pipe 9 and acetone containing 3% by weight of sulfuric acid was supplied into the reaction vessel from pipes 4 and 5 at a rate of 9.5 l/hr. The hydrogen peroxide/alcoholic hydroxyl group molar ratio was 0.74. Reaction was carried out with stirring under atmospheric pressure and reflux of acetone (74°C.). The refluxed acetone was passed through a condenser 6 and a drum 7 at a rate of about 26 Kg/hr and was returned to the reaction vessel from a pipe 8 while diluting hydrogen peroxide fed through the scattering device 12. The liquid reaction mixture was continuously withdrawn from the reaction system by overflowing so that the residence time was 30 minutes. The concentration of hydroquinone (HQ) in the obtained liquid reaction mixture was 12.5% by weight, which corresponded to a yield of 136% based on p-DHP and also to a yield of 95.5% based on the sum of p-DHP and p-HHP. The ratio (%) of effective utilization of hydrogen peroxide, which was calculated according to the following formula:

$$\frac{[(\text{mols of formed HQ}) - (\text{mols of fed p-DHP})]}{(\text{mols of fed } H_2O_2)} \times 100$$

was 100%.

The obtained liquid reaction mixture was neutralized, and acetone, water and MIBK were removed by distillation, and hydroquinone was extracted with water and then extracted with MIBK. The crystalline hydroquinone left after distillation of MIBK was substantially colorless. The absorbance (determined by using a 5-mm cell) of a solution containing 5% by weight of this hydroquinone in MIBK at 420 m$\mu$ was 0.075.

Example 2

Reaction was carried out in the same manner as described in Example 1 except that the feed rates of the respective starting materials were changed as follows:

MIBK solution of oxidation product of p-diisopropylbenzene: 35 l/hr

60% aqueous solution of hydrogen peroxide: 0.67 l/hr (0.83 Kg/hr)

Acetone solution containing 3% of sulfuric acid: 9.7 l/hr

Refluxed acetone: about 33 Kg/hr

Hydrogen peroxide/alcoholic hydroxyl group molar ratio: 0.92

The concentration of hydroquinone in the obtained liquid reaction mixture was 12.9%, which corresponded to a yield of 141% based on p-DHP and also to a yield of 99.0% based on the sum of p-DHP and p-HHP. The ratio of effective utilization of hydrogen peroxide was 90%. The liquid reaction mixture was treated in the same manner as described in Example 1. The crystalline hydroquinone obtained was substantially colorless, and the absorbance of a solution containing 5% by weight of this hydroquinone in MIBK at 420 m$\mu$ was 0.085.

Example 3

Reaction was carried out in the same manner as described in Example 1 except that a reaction vessel having an inner capacity of 50 litres and a structure shown in Fig. 1 was used as the reaction vessel. The concentration of hydroquinone in the obtained liquid reaction mixture was 12.4% by weight, which corresponded to a yield of 135% based on p-DHP and also to a yield of 94.7% based on the sum of p-DHP and p-HHP. The ratio of effective utilisation of hydrogen peroxide was 97%.

Comparative Example 1

A reaction vessel having an inner capacity of 50 litres, which was constructed by modifying the reaction vessel shown in Fig. 2 so that hydrogen peroxide was directly introduced into the reaction mixture from the pipe 9 without passage through the scattering device 12 was used, and reaction was carried out in the same manner as described in Example 1. The concentration of hydroquinone in the obtained liquid reaction mixture was 11.0% by weight, which corresponded to a yield of 120% based

on p-DHP and also to a yield of 84.1% based on the sum of p-DHP and p-HHP. The ratio of effective utilization of hydrogen peroxide was reduced to 55%. The obtained liquid reaction mixture was treated in the same manner as described in Example 1. The crystalline hydroquinone obtained was quite reddish in color, and a 5% by weight solution of this hydroquinone in MIBK had an absorbance as high as 0.37 at 420 mμ.

Comparative Example 2

A reaction vessel having an inner capacity of 50 litres, which was constructed by modifying the reaction vessel shown in Fig. 1 so that the scattering device 10 was removed and the mixture of hydrogen peroxide and refluxed acetone was directly introduced into the reaction mixture from the pipe 8 or 9, was used as the reaction vessel. Reaction was carried out in the same manner as described in Example 1.

The concentration of hydroquinone in the obtained reaction mixture was 11.5% by weight, which corresponded to a yield of 125% based on p-DHP and also to a yield of 87.9% based on the sum of p-DHP and p-HHP. The ratio of effective utilization of hydrogen peroxide was 70%. The crystalline hydroquinone obtained by treating the liquid reaction mixture in the same manner as described in Example 1 had a yellowish red color, and a 5% by weight solution of this hydroquinone in MIBK had an absorbance of 0.21 at 420 mμ.

Example 4

A reaction vessel as shown in Fig. 2, which had an inner capacity of 50 litres, was used. An oxidation product of a mixture of m-cymene and p-cymene, which contained 68.8% by weight of a mixture of m- and p-cymene hydroperoxides, 9.3% by weight of a mixture of m- and p-tertiary alcohols and 2.2% by weight of a mixture of m- and p-primary alcohols, was supplied into the reaction vessel at a rate of 70 l/hr from a perforated plate 2 through a pipe 3. A 60% aqueous solution of hydrogen peroxide was supplied into the reaction vessel at a rate of 1.47 l/hr (1.82 Kg/hr) from a scattering device 12 through a pipe 9, and acetone containing 1.78% by weight of sulfuric acid was supplied into the reaction vessel at a rate of 22 l/hr from pipes 4 and 5. The hydrogen peroxide/tertiary alcohol molar ratio was 0.74. Reaction was carried out with stirring under atmospheric pressure and reflux of acetone (78°C.). The circulation ratio of refluxed acetone was about 70 Kg/hr. The liquid reaction mixture was continuously withdrawn from the reaction system by overflowing so that the residence time was 15 minutes.

The concentration of a mixture of m- and p-cresols in the obtained liquid mixture was 30% by weight, which corresponded to a yield of 86.3% based on the mixture of m- and p-cymene hydroperoxides. The ratio (%) of effective utilization of hydrogen peroxide, which was calculated according to the following formula:

$$\frac{[(\text{mols of formed cresols}) - (\text{mols of fed cymene hydroperoxides})]}{(\text{mols of fed } H_2O_2)} \times 100$$

was 77%.

**Claims**

1. A process for the preparation of phenols by reacting in a reaction vessel (1) an α-hydroxyalkyl-substituted aromatic compound and/or an α,β-unsaturated alkyl-substituted aromatic compound with hydrogen peroxide in the presence of an acid catalyst and, as solvent, acetone, characterised in that the reaction is carried out under such conditions that acetone is distilled off, condensed and recycled to the reaction vessel (1), hydrogen peroxide is diluted with at least 10 times its volume of recycled acetone, and the diluted hydrogen peroxide is scattered into the reaction vessel by sprinkling, the hydrogen peroxide being used in an amount smaller than the amount equimolar to the sum of the amount(s) of secondary alcoholic hydroxyl groups, tertiary alcoholic hydroxyl groups and/or olefinic double bonds in the α-hydroxyalkyl-substituted aromatic compound and/or the α,β-unsaturated alkyl-substituted aromatic compound.

2. A process according to claim 1 characterised by the steps of (i) supplying a starting material containing a hydroperoxide of an isopropyl aromatic compound and an α-hydroxypropyl-substituted aromatic compound and/or α,β-unsaturated isopropenyl-substituted aromatic compound, which has been obtained by oxidizing an isopropyl aromatic compound with molecular oxygen, to a liquid phase reaction mixture in a reaction vessel (1) containing an acid catalyst and acetone by scattering the starting material from a first scattering device (2) located in the gas phase above said reaction mixture, (ii) supplying diluted hydrogen peroxide, which has been formed by diluting hydrogen peroxide with acetone so that the volume is 10 to 100 times the volume of the undiluted hydrogen peroxide, into said reaction mixture by scattering into said reaction mixture from a second scattering device (10 or 12) located in the gas phase above said reaction mixture, (iii) contacting the starting material, hydrogen peroxide and acid catalyst with one another while distilling off acetone from the reaction mixture, (iv)

condensing the distilled acetone and recycling the condensed acetone as a diluent to step (ii), and (v) recovering the resulting phenol from the reaction mixture.

3. A process according to claim 2 wherein said second scattering device (10 or 12) is located at a position higher than the position of the first scattering device (2), and the diluted hydrogen peroxide and the starting material are continuously scattered through said second scattering device (10 or 12) and said first scattering device (2), respectively.

4. A process according to claim 2 or 3 characterised in that there is used as the second scattering device, a scattering device (12) comprising an annular acetone feed pipe (18) having an opening (17) in the lower portion thereof and a plurality of hydrogen peroxide feed nozzles (15), each having a top end communicating with an annular hydrogen peroxide feed pipe (14) and a discharge end located substantially at the same position as said opening 17, whereby hydrogen peroxide falling down from said nozzles (15) is diluted with acetone.

5. A process according to claim 2, 3 or 4 characterised in that the isopropyl aromatic compound is diisopropylbenzene or cymene.

6. An apparatus for use in the preparation of phenols which comprises a reaction vessel (1), an annular diluent feed pipe (18) having an opening (17) in the lower portion thereof and a plurality of hydrogen peroxide feed nozzles (15), each nozzle having a discharge end located at substantially the same position as said opening (17), said diluent feed pipe (18) and hydrogen peroxide feed nozzles (15) being disposed in an upper portion of the reaction vessel (1).

**Patentansprüche**

1. Verfahren zur Herstellung von Phenolen durch Umsetzung einer $\alpha$-hydroxyalkyl-substituierten aromatischen Verbindung und/oder einer $\alpha,\beta$-ungesättigten alkyl-substituierten aromatischen Verbindung mit Wasserstoffperoxid in Gegenwart eines Säurekatalysators und mit Aceton als Lösungsmittel in einem Reaktionsgefäß (1), dadurch gekennzeichnet, daß die Reaktion unter derartigen Bedingungen geführt wird, daß Aceton abdestilliert, kondensiert und zum Reaktionsgefäß (1) zurückgeführt wird, Wasserstoffperoxid mit mindestens dem Zehnfachen seines Volumens an rückgeführtem Aceton verdünnt wird, und das verdünnte Wasserstoffperoxid durch Sprühen in das Reaktionsgefäß hinein verteilt wird, wobei das Wasserstoffperoxid in einer Menge eingesetzt wird, die geringer ist als die Menge, die der Summe der Mengen von sekundären alkoholischen Hydroxylgruppen, tertiären alkoholischen Hydroxylgruppen und/oder olefinischen Doppelbindungen in der $\alpha$-hydroxyalkyl-substituierten aromatischen Verbindung und/oder der $\alpha,\beta$-ungesättigten alkyl-substituierten aromatischen Verbindung äquimolar ist.

2. Verfahren nach Anspruch 1, dadurch gekennzeichnet, daß in einem Schritt (i) ein Ausgangsmaterial, welches ein Hydroperoxid einer Isopropyl-Aromatenverbindung und eine $\alpha$-hydroxypropyl-substituierte aromatische Verbindung und/oder eine $\alpha,\beta$-ungesättigte isopropyl-substituierte aromatische Verbindung, die durch Oxidation eines Isopropylaromaten mit molekularem Sauerstoff erhalten wurde, aufweist, einem Flüssigphasen-Reaktionsgemisch in einem Reaktionsgefäß (1) zugeführt wird, welches einen Säurekatalysator und Aceton durch Verteilung des Ausgangsmaterials aus einer ersten Zerstreu-Vorrichtung (2), die sich in der Gasphase über dem Reaktionsgemisch befindet, aufweist, (ii) verdünntes Wasserstoffperoxid, welches durch Verdünnen von Wasserstoffperoxid mit Aceton, so daß das Volumen 10 bis 100-mal dem Volumen des unverdünnten Wasserstoffperoxids entspricht, erhalten wurde, dem Reaktionsgemisch zugeführt wird, indem es in das Reaktionsgemisch von einer zweiten Zerstreu-Vorrichtung (10 oder 12), die sich in der Gasphase über dem Reaktionsgemisch befindet, hinein verteilt wird, (iii) das Ausgangsmaterial, Wasserstoffperoxid und Säurekatalysator miteinander in Berührung gebracht werden, während Aceton aus dem Reaktionsgemisch abdestilliert wird, (iv) das destillierte Aceton kondensiert und das kondensierte Aceton als Verdünnungsmittel zum Schritt (ii) zurückgeführt wird, und (v) das erhaltene Phenol aus dem Reaktionsgemisch gewonnen wird.

3. Verfahren nach Anspruch 2, dadurch gekennzeichnet, daß die zweite Zerstreu-Vorrichtung (10 oder 12) in einer höheren Lage als die Lage der ersten Zerstreu-Vorrichtung (2) angeordnet ist, und das verdünnte Wasserstoffperoxid und das Ausgangsmaterial kontinuierlich durch die zweite Zerstreu-Vorrichtung (10 oder 12) bzw. durch die erste Zerstreu-Vorrichtung (2) hindurch verteilt werden.

4. Verfahren nach Anspruch 2 oder 3, dadurch gekennzeichnet, daß als zweite Zerstreu-Vorrichtung eine Zerstreu-Vorrichtung (12) verwendet wird, die ein ringförmiges Aceton-Zuflußrohr (18) mit einer Öffnung (17) im unteren Teil und einer Vielzahl von Wasserstoffperoxid-Zuführungsdüsen (15) mit jeweils einem oberen Ende, welches mit einem ringförmigen Wasserstoffperoxid-Zuflußrohr (14) in Verbindung steht und einem Auslaß, der sich an etwa der gleichen Stelle wie die Öffnung (17) befindet, wodurch das von den Düsen (15) niederfallende Wasserstoffperoxid mit Aceton verdünnt wird, aufweist.

5. Verfahren nach Anspruch 2, 3 oder 4, dadurch gekennzeichnet, daß die isopropylaromatische Verbindung Diisopropylbenzol oder Zymol ist.

6. Vorrichtung zur Verwendung bei der Herstellung von Phenolen, welche ein Reaktionsgefäß (1), ein ringförmiges Verdünnungsmittel-Zuflußrohr (18) mit einer Öffnung (17) im unteren Teil und einer Vielzahl von Wasserstoffperoxid-Zuführungsdüsen (15), wobei jede Düse einen Auslaß besitzt, der sich

an etwa der gleichen Stelle wie die Offnung (17) befindet, und das Verdünnungsmittel-Zuflußrohr (18) und die Wasserstoffperoxid-Zuführungsdüsen (15) in einem oberen Teil des Reaktionsgefäßes (1) angeordnet sind, umfaßt.

## Revendications

1. Procédé de préparation de phénols par réaction dans un réacteur (1) d'un composé aromatique substitué par un $\alpha$-hydroxyalkyle et/ou d'un composé aromatique substitué par un alkyle $\alpha,\beta$-insaturé avec l'eau oxygénée en présence d'un catalyseur acide et d'acétone comme solvant, caractérisé par le fait que la réaction est effectuée dans des conditions telles que l'acétone est distillée, condensée et recyclée vers le réacteur (1), l'eau oxygénée est diluée avec au moins 10 fois son volume d'acétone recyclée, et l'eau oxygénée diluée est dispersée dans le réacteur par aspersion, l'eau oxygénée étant utilisée en proportion plus faible que la proportion équimolaire par rapport à la somme des proportions de groupements hydroxyle alcools secondaires, de groupements hydroxyle alcools tertiaires et/ou des doubles liaisons oléfiniques dans le composé aromatique substitué par un $\alpha$-hydroxyalkyle et/ou dans le composé aromatique substitué par un alkyle $\alpha,\beta$-insaturé.

2. Procédé selon la revendication 1, caractérisé par le fait qu'il comprend les étapes consistant (i) à ajouter un matériau de départ contenant un hydroperoxyde d'un composé aromatique isopropylé et un composé aromatique substitué par un $\alpha$-hydroxypropyle et/ou un composé aromatique substitué par un isopropènyle $\alpha,\beta$-insaturé, qui a été obtenu par oxydation d'un composé aromatique isopropylé avec de l'oxygène moléculaire, à un mélange réactionnel liquide dans un réacteur (1) contenant un catalyseur acide et de l'acétone en dispersant le matériau de départ depuis un premier dispositif de dispersion (2) situé dans la phase gazeuse au-dessus dudit mélange réactionnel, (ii) à ajouter de l'eau oxygénée diluée, qui a été préparée en diluant l'eau oxygénée avec l'acétone de façon que le volume obtenu soit 10 à 100 fois le volume de l'eau oxygénée non diluée, dans ledit mélange réactionnel par dispersion dans ledit mélange depuis un second dispositif de dispersion (10 ou 12) situé dans la phase gazeuse au-dessus dudit mélange réactionnel, (iii) à mettre en contact le matériau de départ, l'eau oxygénée et le catalyseur acide tout en distillant l'acétone du mélange réactionnel, (iv) à condenser l'acétone distillée et à recycler l'acétone condensée comme diluant pour l'étape (ii), et (v) à séparer le phénol résultant du mélange réactionnel.

3. Procédé selon la revendication 2, dans lequel ledit second dispositif de dispersion (10 ou 12) est situé dans une position plus élevée que la position du premier dispositif de dispersion (2), et dans lequel l'eau oxygénée diluée et le matériau de départ sont dispersés de façon continue à l'aide dudit second dispositif de dispersion (10 ou 12) et dudit premier dispositif de dispersion (2), respectivement.

4. Procédé selon la revendication 2 ou 3, caractérisé par le fait que l'on utilise comme second dispositif de dispersion un dispositif de dispersion (12) comprenant un tuyau annulaire (18) d'alimentation d'acétone ayant une ouverture (17) dans sa partie inférieure et une pluralité de buses d'alimentation (15) en eau oxygénée ayant chacune une extrémité supérieure communiquant avec un tuyau annulaire (14) d'alimentation en eau oxygénée et une extrémité de décharge située sensiblement au même niveau que ladite ouverture (17), grâce à quoi l'eau oxygénée tombant desdites buses (15) est diluée avec l'acétone.

5. Procédé selon la revendication 2, 3 ou 4, caractérisé en ce que le composé aromatique isopropylé est le diisopropylbenzène ou le cymène.

6. Appareil utilisable dans la préparation de phénol qui comprend un réacteur (1), un tuyau annulaire (18) d'alimentation en diluant ayant une ouverture (17) dans sa partie inférieure et une pluralité de buses d'alimentation (15) en eau oxygénée, chaque buse ayant une extrémité de décharge située sensiblement au même niveau que ladite ouverture (17), ledit tuyau (18) d'alimentation en diluant et lesdites buses d'alimentation (15) en eau oxygénée étant disposés dans la partie supérieure du réacteur (1).

*Fig. 1*

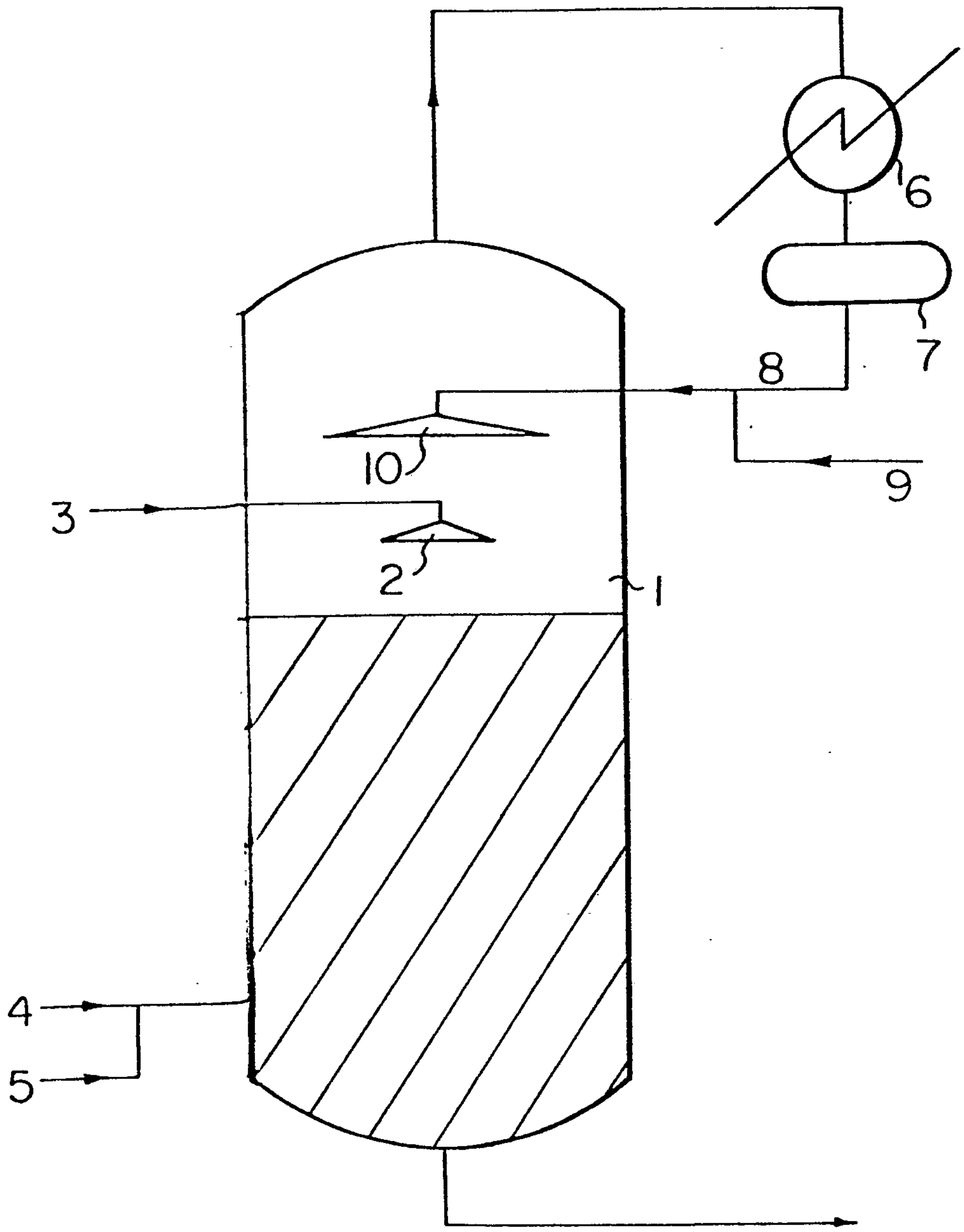

Fig. 2

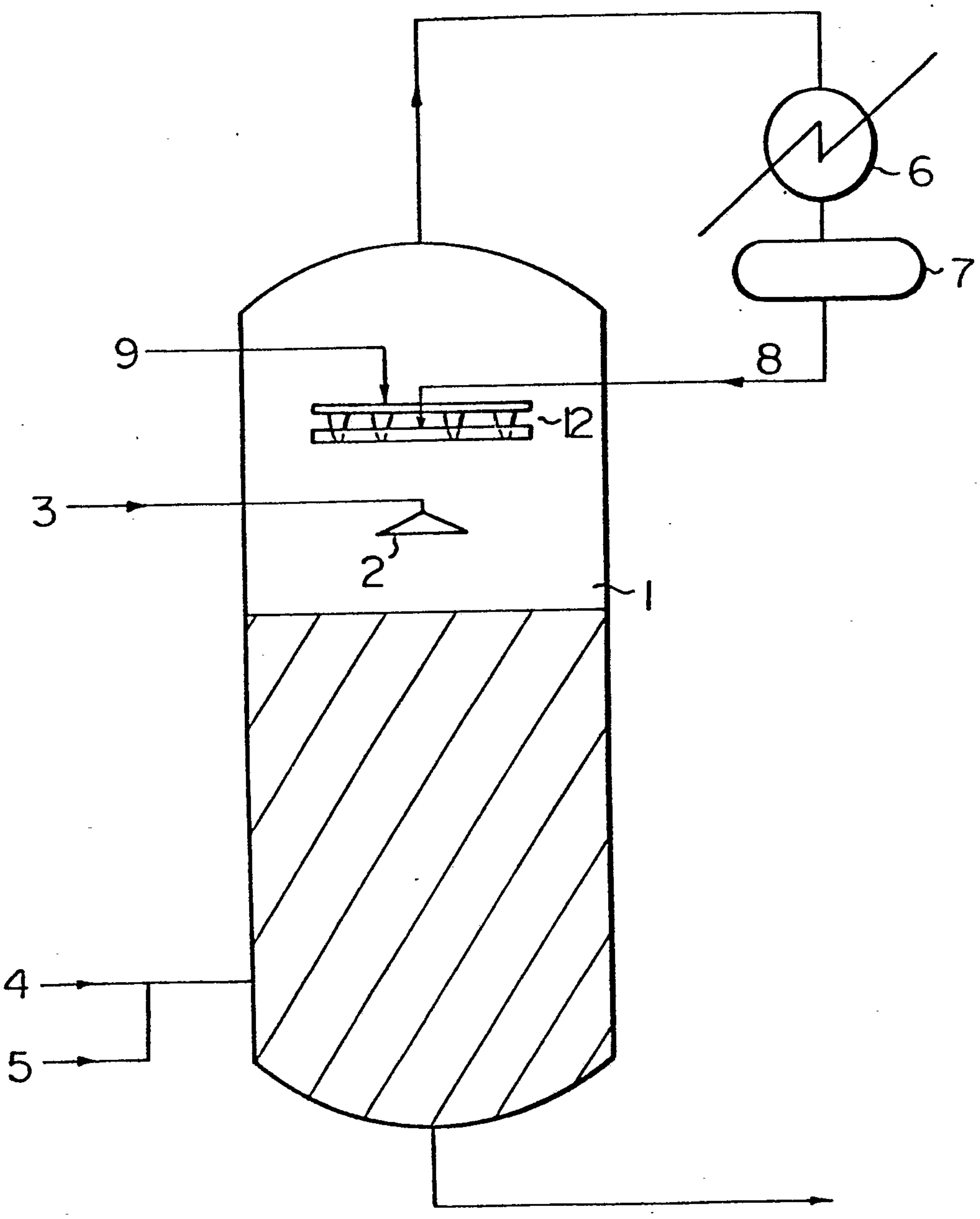

Fig. 3

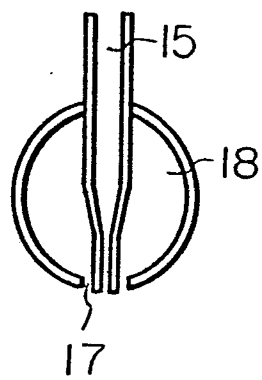


Fig. 4